(19) Europäisches Patentamt
European Patent Office
Office européen des brevets

(11) Publication number: 0 081 793 B1

(12) # EUROPEAN PATENT SPECIFICATION

(45) Date of publication of patent specification: 09.04.86

(51) Int. Cl.$^4$: C 07 D 319/02, A 61 K 31/335

(21) Application number: 82111302.4

(22) Date of filing: 07.12.82

(54) **Novel vitamin D3 derivatives and process for producing the same.**

(30) Priority: 08.12.81 JP 196251/81
17.03.82 JP 40903/82

(43) Date of publication of application:
22.06.83 Bulletin 83/25

(45) Publication of the grant of the patent:
09.04.86 Bulletin 86/15

(84) Designated Contracting States:
AT BE CH DE FR GB IT LI NL SE

(56) References cited:
CHEMICAL ABSTRACTS, vol. 95, no. 23, 7th December 1981, page 718, no. 204296x, Columbus Ohio (USA);

CHEMICAL ABSTRACTS, vol. 93, no. 9, 1st September 1980, page 669, no. 95483s, Columbus Ohio (USA); S. YAMADA et al.: "Synthesis, reaction and biological activity of 6,19-epidioxyvitamin D derivatives derived from vitamin D by dye sensitized photo-oxidation"

(73) Proprietor: Chugai Seiyaku Kabushiki Kaisha
5-1, 5-chome, Ukima Kita-ku
Tokyo (JP)

(72) Inventor: Takayama, Hiroaki
2-6-12-31, Hatagaya
Shibuya-ku Tokyo (JP)
Inventor: Yamada, Sachiko
1227-4-1218, Hatsuzawamachi
Hachioji-shi Tokyo (JP)
Inventor: Nakayama, Keiko
4-4-15, Sennin-cho
Hachioji-shi Tokyo (JP)
Inventor: Suda, Tatsuo
1-8-5, Wakaba-cho Tachikawa-shi
Tokyo (JP)

(74) Representative: Vossius Vossius Tauchner
Heunemann Rauh
Siebertstrasse 4 P.O. Box 86 07 67
D-8000 München 86 (DE)

(56) References cited:
CHEMICAL ABSTRACTS, vol. 94, no. 1, 5th January 1981, page 376, no. 4023y, Columbus Ohio (USA);

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 081 793 B1

**Description**

*Field of the Invention:*

The invention relates to 6,19-epidioxyvitamin $D_3$ derivatives of formula (I):

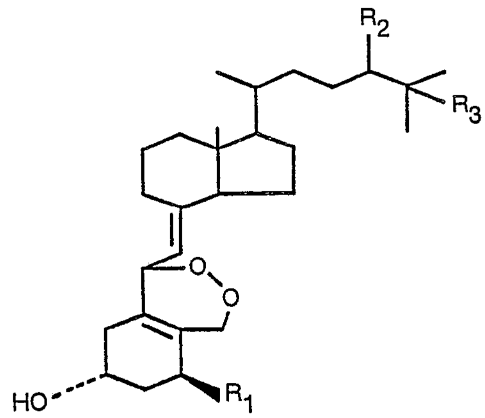

(I)

wherein $R_1$, $R_2$ and $R_3$ are each a hydrogen atom or a hydroxyl group; when $R_1$ is a hydrogen atom, $R_2$ represents a hydroxyl group and $R_3$ is a hydrogen atom or a hydroxyl group; when both $R_1$ and $R_2$ represent a hydroxyl group, $R_3$ is a hydrogen atom or a hydroxyl group; and when $R_1$ is a hydroxyl group and $R_2$ is a hydrogen atom, $R_3$ represents a hydroxyl group.

*Background of the Invention:*

Chem. Abstr. *93* (1980), page 669, No. 95483s discloses 6,19-epidioxyvitamin $D_3$-derivatives with a significant effect on Ca-transport.

As a result of the studies by DeLuca and Kodicek on the separation and identification of the metabolites of vitamin $D_3$ and its metabolism, it has been established that vitamin $D_3$ is first hydroxylated (at 25-position) in the liver to form 25-hydroxyvitamin $D_3$, then is hydroxylated (at 24R-position or at 1α-position) in the kidney to form 1α-25-dihydroxyvitamin $D_3$ and 24R,25-dihydroxyvitamin $D_3$ having hormone activity. It is also well known that these metabolites and other synthetic analogs such as 1α-hydroxyvitamin $D_3$ thereof enhance intestinal calcium transport and bone mineral mobilization and are useful as therapeutic agents to treat the diseases caused by various disorders in calcium metabolism.

A recent study based on experiments with a myeloid leukemia cell line (M1) isolated from an SL mouse with myeloid leukemia has revealed that the above named vitamin $D_3$ derivatives can induce differentiation of myeloid leukemia cells and that they are at least about 100 times as potent as dexamethasone the most potent inducer ever known [Proc. Natl. Acad. Sci. U.S.A. *78*, 4990 (1981)]. Sachs [Brit. J. Haematol., *40*, 509 (1978)] and Hozumi ["Gan to Kagaku-ryoho (Cancer and Chemotherapy)", *8*(1), 9, (1981)] have suggested that compounds capable of inducing differentiation of myeloid leukemia cells can be used to treat leukemia and that use of these compounds is promising as a supplement to the conventional chemotherapy and immunotherapy. By having a very great ability to induce differentiation, these derivatives also have significant effects on calcium metabolism in vivo, and an overdose of them may cause hypercalcemia. So, the derivatives are not completely satisfactory for use as a drug to treat leukemia which sometimes requires continuous administration of the drug in high dose.

*Summary of the Invention:*

The inventors have made various studies on vitamin $D_3$ derivatives and have found that 6,19-epidioxyvitamin $D_3$ derivatives are highly capable of inducing differentiation of human myeloid leukemia cells with minimum effects on calcium metabolism and that hence, they are useful as an agent to treat leukemia.

*Detailed Description of the Invention:*

The ability of the compounds (I) of the invention to induce differentiation of human myeloid leukemia cells was determined by the following method.

HL—60 cells (human myeloid leukemia cell line) were cultured at 37°C in RPMI 1640 medium, GIBCO, Grand Island, NY) supplemented with 10% heat-inactivated fetal calf serum (Flow Laboratories, Rockville, MD) and 100 U/ml of penicillin and 100 μg/ml of streptomycin in a humidified atmosphere of 5% $CO_2$ in air. Cells were inoculated at $1 \times 10^5$ cells/ml and incubated with various concentrations of inducing compounds. The differentiation-associated properties were tested 3 days after inoculation. The formation of Fc and C3 rosettes was assayed according to the method of Lotem and Sachs Int. J. Cancer *15*, 731 (1975) using sheep erthrocytes coated with rabbit anti-sheep erythrocytes antibody, or with the antibody and mouse complement, respectively. The percentage of cells with a rosette to which at least 5 erythrocytes were bound was counted with a hemocytometer. At least 200 cells were counted. Phagocytic activity was

measured according to the method of Collins *et al.* Proc. natl. Acad. Sci. U.S.A. *75*, 2458 (1978). Cells were suspended at a concentration of 2 × $10^6$ cells/ml in RPMI 1640 medium supplemented with 10% fetal calf serum and 10% human AB serum. *Candida albicans* were washed with saline and added to the cell suspension at a final concentration of 4 × $10^6$/ml. The suspension was incubated at 37°C for 30 min. The percentage of cells that had phagocytosed at least one *Candida* was counted with a hemocytometer.

The results of the experiment are shown in the following table, wherein Ia to If for the chemicals' names are keyed to the symbols used in Examples 1 to 3.

TABLE

| Chemicals added | Concentration (ng/ml) | Phagocytic Cells (%) | C3 receptor (%) |
|---|---|---|---|
| control | — | 3.8 | 9.9 |
| Ia | 25 | 14.9 | 14.4 |
| | 250 | 46.5 | 37.4 |
| Ib | 25 | 20.0 | 20.8 |
| | 250 | 42.7 | 40.0 |
| Ic | 25 | 20.0 | 22.7 |
| | 250 | 47.5 | 41.8 |
| Id | 25 | 14.9 | 16.5 |
| | 250 | 38.0 | 42.6 |
| Ie | 50 | 28.0 | 28.0 |
| | 500 | 60.3 | 47.1 |
| If | 50 | 16.0 | 23.9 |
| | 500 | 54.3 | 46.3 |

The influence of the compounds of this invention upon calcium metabolism was determined by the following Experiment.

Experiment

Weanling Sprague Dawley male rats weighing 45 to 50 g were fed on Diet 11 and deionized water under an incandescent lamp for a period of 6 weeks. They were administered orally a solution of each compound in MCT (medium chain triglyeride of The Nisshin Oil Mills, Ltd.) once a day. The animals were starved, beheaded and bled to obtain blood samples. The duodenum was removed from each animal and checked for $^{45}Ca$ absorption by the inverted intestinal tract method [Amer. J. Physics., *216*, 1351 (1969)]. Plasma was separated from the blood samples and the contents of calcium and inorganic phosphorus were determined by the OCPC method [Am. J. Clin. Path., *45*, 290 (1966)] and the method of Peel and Loughman [Biochem. J., *65*, 709 (1957)], respectively. The compounds of the invention were far less capable of transporting calcium from the intestinal tract than known vitamin $D_3$ compounds such as 1α-hydroxyvitamin $D_3$ and 1α,25-dihydroxyvitamin $D_3$. The compounds had also a very weak effect on the concentrations of phosphorus and calcium in plasma.

The compounds of formula (I) of the invention are novel and they include 6,19-epidioxy-9,10-secocholesta-5(10),7-dien-3β-24-diol, 6,19-epidioxy-9,10-secocholesta-5(10),7-dien-1α,3β,24-triol, 6,19-epidioxy-9,10-secocholesta-5(10),7-dien-1α,3β,24,25-tetraol and 6,19-epidioxy-9,10-secocholesta-5(10),7-dien-1α,3β-25-triol. These compounds can be prepared by subjecting corresponding vitamin $D_3$ compounds, say 24-hydroxyvitamin $D_3$, 1α,24-dihydroxyvitamin $D_3$, 1α,24,25-trihydroxyvitamin $D_3$ and 1α,25-dihydroxyvitamin $D_3$ to photosensitized oxidation. This can be done by illuminating the corresponding vitamin $D_3$ compounds with visible light emitting sources such as a halogen lamp or tungsten lamp in oxygen or air as they are dissolved in an inert organic solvent such as methanol, ethanol or propanol in the presence of an organic pigment such as Rose Bengal, Eosine or methylene blue. The illumination period preferably continues until the vitamin $D_3$ compounds are no longer present in the reaction system. The compounds (I)

can be isolated from the reaction mixture by a conventional method, such as distilling off the solvent followed by column chromatography. The end compounds (I) have an asymmetric carbon atom at 6- or 24-position, and in each case, they include 2 to 4 optical isomers.

The invention is now described in greater detail by reference to the following examples which are given here for illustrative purposes only and are by no means intended to limit its scope.

Example 1

A mixture of 24R-hydroxyvitamin $D_3$ (2.0 mg) and Rose Bengal (10 mg) was dissolved in a mixed solvent consisting of benzene (24 mg) and ethanol (6 ml), and after bubbling oxygen the solution was illuminated with a halogen lamp (200 W) for 40 minutes under cooling with ice. Argon was passed through the reaction mixture, which was subsequently washed with water, dried on sodium sulfate and had the solvent distilled off. The residue was subjected to column chromatography (solvent: 20% ethyl acetate-benzene) on silica gel, whereupon (6R,24R)-6,19-epidioxy-9,10-secocholesta-5(10),7-dien-3β,24-diol (Ia) and (6S,24R)-6,19-epidioxy-9,10-secocholesta-5(10),7-dien-3β,24-diol (Ib) were obtained.

(6R,24R)-form (Ia)

NMR ($CDCl_3$) δ:

0.59 (3H, s), 3.36 (1H, m), 4.12 (1H, m), 4.40 (1H, d, J=16H), 4.48 (1H, d, J=16Hz), 4.94 (1H, d, J=9Hz), 5.22 (1H, d, J=9Hz)

Mass m/e:

432 ($M^+$), 414, 396, 287, 285, 151

(6S,24)-form (Ib)

NMR ($CDCl_3$) δ:

0.59 (3H, s), 3.34 (1H, m), 3.97 (1H, m), 4.22 (1H, d, J=16Hz), 4.64 (1H, d, J=16Hz), 4.83 (1H, d, J=9Hz), 5.27 (1H, d, J=9Hz)

Mass m/e:

432 ($M^+$), 414, 396, 287, 285, 151

Example 2

A mixture of 1α,24R-dihydroxyvitamin $D_3$ (660 μg) and Rose Bengal (20 mg) was dissolved in ethanol (15 ml), and after bubbling oxygen the solution was illuminated with a halogen lamp (200 W) for one hour under cooling with ice. Argon was passed through the reaction mixture, ethanol distilled off and the residue was dissolved in ethyl acetate. The solution was washed with water, the ethyl acetate layer dried over sodium sulfate and the solvent was distilled off. The residue was subjected to column chromatography (solvent: hexane/chloroform/methanol = 30/70/3) on Sephadex LH—20 (6 g). Fractions 22 to 30 (3 g for each fraction) were concentrated into 358 g of a residue. The residue was subjected to highpressure liquid chromatography (column: Lichrosorb, eluant: 20% isopropanol-hexane), whereupon two isomers with different degrees of polarity of (24R)-6,19-epidioxy-9,10-secochlesta-5(10),7-dien-1α,3β,24-triol having different configurations at 6-position, were obtained, and the yield of one compound (Ic) having a weaker polarity was 78 μg and that of the other compound (Id) having a stronger polarity was 310 μg.

Mass spectra for the two compounds

m/3: 430 (M—18), 412, 394, 379

Example 3

A mixture of 1α,25-dihydroxyvitamin $D_3$ (840 μg) and Rose Bengal (20 mg) was dissolved in 15 ml of ethanol (J. P. 10th ed.), and after bubbling oxygen the solution was illuminated with a halogen lamp (200 W) for 1.5 hours. The solvent was distilled off under vacuum and the residue was purified by column chromatography on silica gel, whereupon two isomers with different degrees of polarity and having different configurations at 6-position were obtained, and the yield of one compound (Ie) having a weaker polarity was 150 μg and that of the other compound (If) having a stronger polarity was 310 μg.

Data for compound Ie

Mass m/e:

430 ($M^+$—18), 412, 394, 379

UV (95% ethanol):

no absorption maximum beyond 210 nm

Data for compound If

Mass m/e:

430 ($M^+$—18), 412, 394, 379

UV (95% ethanol):

no absorption maximum beyond 210 nm

**Claims for the Contracting States: BE CH DE FR GB IT LI NL SE**

1. A 6,19-epidioxyvitamin $D_3$ derivative of the formula (I):

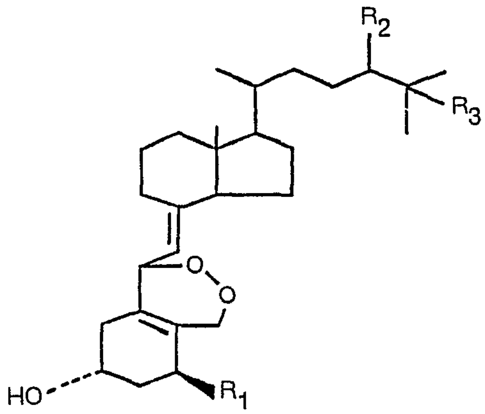

(I)

wherein $R_1$, $R_2$ and $R_3$ are each a hydrogen atom or a hydroxyl group; when $R_1$ is a hydrogen atom, $R_2$ represents a hydroxyl group and $R_3$ is a hydrogen atom or a hydroxyl group; when both $R_1$ and $R_2$ represent a hydroxyl group, $R_3$ is a hydrogen atom or a hydroxyl group; and when $R_1$ is a hydroxyl group and $R_2$ is a hydrogen atom, $R_3$ represents a hydroxyl group.

2. A process for producing a 6,19-epidioxyvitamin $D_3$ derivative of the formula (I) of Claim 1 by subjecting a vitamin $D_3$ compound of the formula (II):

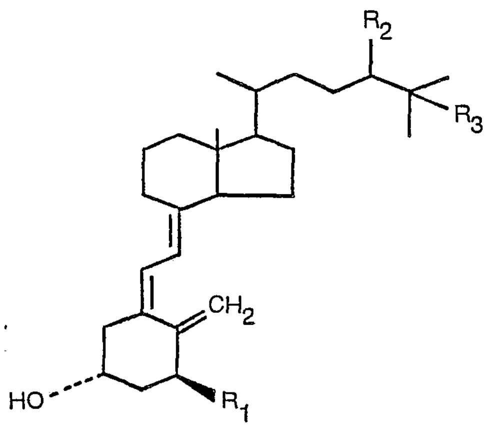

(II)

wherein $R_1$, $R_2$ and $R_3$ are the same as defined above to photo-oxidation in an inert organic solvent.

3. A process according to Claim 2 wherein a photosensitizer such as Rose Bengal, Eosine or methylene blue is used.

4. A process according to Claim 2 or 3 wherein the vitamin $D_3$ compound is illuminated with visible light from a halogen lamp or tungsten lamp in the presence of air or oxygen.

5. A compound according to Claim 1 wherein the 6,19-epidioxyvitamin $D_3$ derivative is 6,19-epidioxy-9,10-secocholesta-5(10),7-dien-3β,24-diol.

6. A compound according to Claim 1 wherein the 6,19-epidioxyvitamin $D_3$ derivative is 6,19-epidioxy-9,10-secocholesta-5(10),7-dien-1α,3β,24-triol.

7. A compound according to Claim 1 wherein the 6,19-epidioxyvitamin $D_3$ derivative is 6,19-epidioxy-9,10-secocholesta-5(10),7-dien-1α,3β,25-triol.

8. Pharmaceutical composition comprising at least one of the compounds as set forth in claims 1 to 7.

**Claims for the Contracting State: AT**

1. A process for producing a 6,19-epidioxyvitamin $D_3$ derivative of the formula (I):

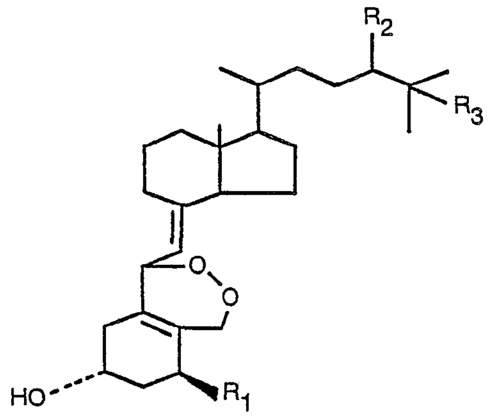

(I)

wherein $R_1$, $R_2$ and $R_3$ are each a hydrogen atom or a hydroxyl group; when $R_1$ is a hydrogen atom, $R_2$ represents a hydroxyl group and $R_3$ is a hydrogen atom or a hydroxyl group; when both $R_1$ and $R_2$ represent a hydroxyl group, $R_3$ is a hydrogen atom or a hydroxyl group; and when $R_1$ is a hydroxyl group and $R_2$ is a hydrogen atom, $R_3$ represents a hydroxyl group by subjecting a vitamin $D_3$ compound of the formula (II):

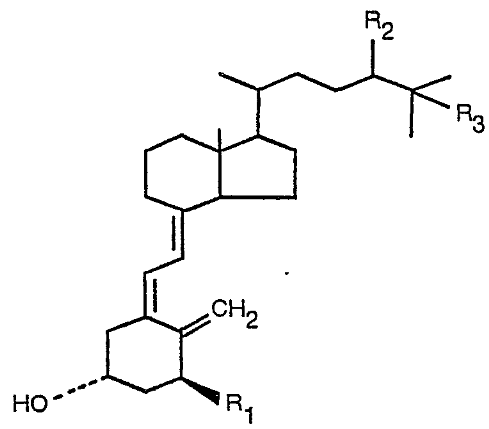

(II)

wherein $R_1$, $R_2$ and $R_3$ are the same as defined above to photo-oxidation in an inert organic solvent.

2. A process according to Claim 1 wherein a photosensitizer such as Rose Bengal, Eosine or methylene blue is used.

3. A process according to Claim 1 or 2 wherein the vitamin $D_3$ compound is illuminated with visible light from a halogen lamp or tungsten lamp in the presence of air or oxygen.

4. A process for producing a compound according to Claim 1 wherein the 6,19-epidioxyvitamin $D_3$ derivative is 6,19-epidioxy-9,10-secocholesta-5(10),7-dien-3β,24-diol.

5. A process for producing a compound according to Claim 1 wherein the 6,19-epidioxyvitamin $D_3$ derivative is 6,19-epidioxy-9,10-secocholesta-5(10),7-dien-1α,3β,24-triol.

6. A process for producing a compound according to Claim 1 wherein the 6,19-epidioxyvitamin $D_3$ derivative is 6,19-epidioxy-9,10-secocholesta-5(10),7-dien-1α,3β,25-triol.

7. A process for producing a pharmaceutical composition comprising the formulation of at least one of the compounds as set forth in claims 1 to 6.

**Patentansprüche für die Vertragsstaaten: BE CH DE FR GB IT LI NL SE**

1. 6,19-Epidioxyvitamin $D_3$-Derivat der Formel (I)

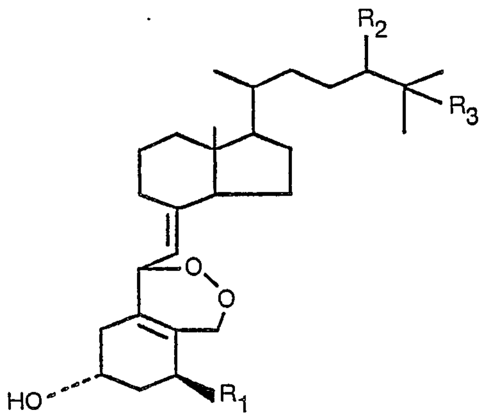

(I)

in der $R_1$, $R_2$ und $R_3$ jeweils ein Wasserstoffatom oder eine Hydroxylgruppe bedeuten, wenn $R_1$ ein Wasserstoffatom ist, $R_2$ eine Hydroxylgruppe und $R_3$ ein Wasserstoffatom oder eine Hydroxylgruppe bedeuten, wenn beide Reste $R_1$ und $R_2$ eine Hydroxylgruppe bedeuten, $R_3$ ein Wasserstoffatom oder eine Hydroxylgruppe ist, und wenn $R_1$ eine Hydroxylgruppe bedeutet und $R_2$ ein Wasserstoffatom darstellt, $R_3$ eine Hydroxylgruppe bedeutet.

2. Verfahren zur Herstellung eines 6, 19-Epidioxyvitamin $D_3$-Derivates der Formel (I) nach Anspruch 1 durch Photooxidation einer Vitamin $D_3$-Verbindung der Formel (II)

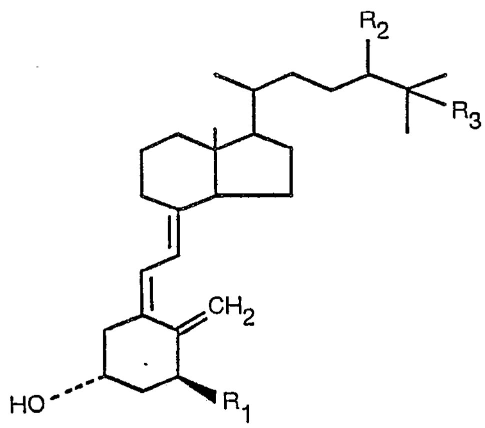

(II)

in der $R_1$, $R_2$ und $R_3$ wie vorstehend definiert sind, in einem inerten organischen Lösungsmittel.

3. Verfahren nach Anspruch 2, wobei ein Photosensibilisator, wie Rose Bengal, Eosin oder Methylenblau verwendet wird.

4. Verfahren nach Anspruch 2 oder 3, wobei die Vitamin $D_3$-Verbindung mit sichtbarem Licht aus einer Halogenlampe oder Wolframlampe in Gegenwart von Luft oder Sauerstoff belichtet wird.

5. Verfahren nach Anspruch 1, wobei das 6,19-Epidioxyvitamin $D_3$-Derivat 6,19-Epidioxy-9,10-secocholesta-5(10),7-dien-3β,24-diol ist.

6. Verbindung nach Anspruch 1, wobei das 6,19-Epidioxyvitamin $D_3$-Derivat 6,19-Epidioxy-9,10-secocholesta-5(10),7-dien-1α,3β,24-triol ist.

7. Verbindung nach Anspruch 1, wobei das 6,19-Epidioxyvitamin $D_3$-Derivat 6,19-Epidioxy-9,10-secocholesta-5(10),7-dien-1α,3β,25-triol ist.

8. Arzneimittel, enthaltend mindestens eine der Verbindungen nach den Ansprüchen 1 bis 7.

**Patentansprüche für den Vertragsstaat: AT**

1. Verfahren zur Herstellung eines 6,19-Epidioxyvitamin $D_3$-Derivates der Formel (I)

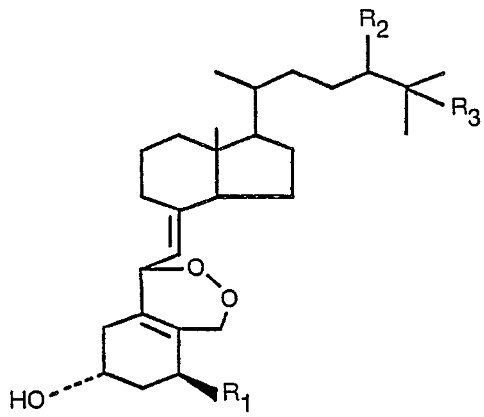

(I)

in der $R_1$, $R_2$ und $R_3$ jeweils ein Wasserstoffatom oder eine Hydroxylgruppe bedeuten, wenn $R_1$ ein Wasserstoffatom ist, $R_2$ eine Hydroxylgruppe und $R_3$ ein Wasserstoffatom oder eine Hydroxylgruppe bedeuten, wenn beide Reste $R_1$ und $R_2$ eine Hydroxylgruppe bedeuten, $R_3$ ein Wasserstoffatom oder eine Hydroxylgruppe ist, und wenn $R_1$ eine Hydroxylgruppe bedeutet und $R_2$ ein Wasserstoffatom darstellt, $R_3$ eine Hydroxylgruppe bedeutet, durch Photooxiidation einer Vitamin $D_3$-Verbindung der Formel (II)

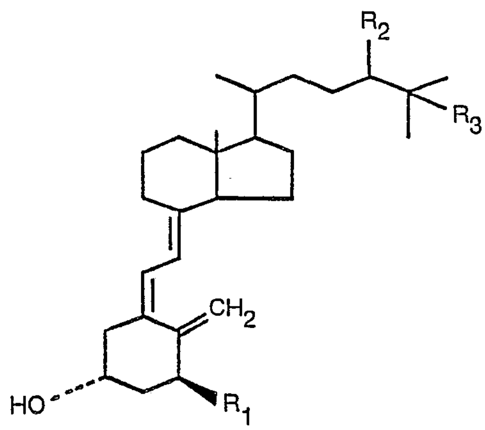

(II)

in der $R_1$, $R_2$ und $R_3$ wie vorstehend definiert sind, in einem inerten organischen Lösungsmittel.

2. Verfahren nach Anspruch 1, wobei ein Photosensibilisator, wie Rose Bengal, Eosin oder Methylenblau verwendet wird.

3. Verfahren nach Anspruch 1 oder 2, wobei die Vitamin $D_3$-Verbindung mit sichtbarem Licht aus einer Halogenlampe oder Wolframlampe in Gegenwart von Luft oder Sauerstoff belichtet wird.

4. Verfahren zur Herstellung einer Verbindung nach Anspruch 1, wobei das 6,19-Epidioxyvitamin $D_3$-Derivat 6,19-Epidioxy-9,10-secocholesta-5(10),7-dien-3β,24-diol ist.

5. Verfahren zur Herstellung einer Verbindung nach Anspruch 1, wobei das 6,19-Epidioxyvitamin $D_3$-Derivat 6,19-Epidioxy-9,10-secocholesta-5(10),7-dien-1α,3β,24-triol ist.

6. Verfahren zur Herstellung einer Verbindung nach Anspruch 1, wobei das 6,19-Epidioxyvitamin $D_3$-Derivat 6,19-Epidioxy-9,10-secocholesta-5(10),7-dien-1α,3β,25-triol ist.

7. Verfahren zur Herstellung eines Arzneimittels, umfassend die Formulierung mindestens einer Verbindung nach den Ansprüchen 1 bis 6.

**Revendications pour les Etats contractants: BE CH DE FR GB IT LI NL SE**

1. Dérivé de 6,19-épidioxyvitamine D3 de formule (I)

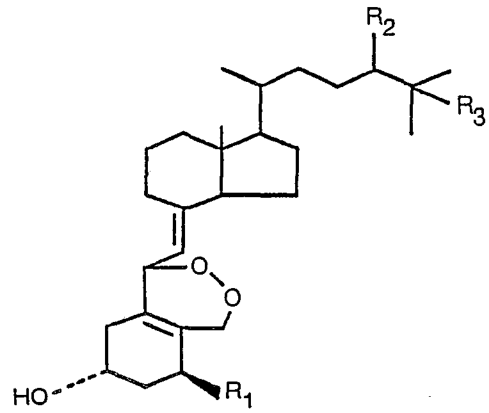

(I)

dans laquelle $R_1$, $R_2$ et $R_3$ sont chacun un atome d'hydrogène ou un groupe hydroxyle; lorsque $R_1$ est un atome d'hydrogène, $R_2$ représente un groupe hydroxyle et $R_3$ est un atome d'hydrogène ou un groupe hydroxyle; lorsque $R_1$ et $R_2$ représentent chacun un groupe hydroxyle, $R_3$ est un atome d'hydrogène ou un groupe hydroxyle; et lorsque $R_1$ est un groupe hydroxyle et $R_2$ est un atome d'hydrogène, $R_3$ représente un groupe hydroxyle.

2. Procédé pour la préparation d'un dérivé de 6,19-épidioxyvitamine D3 de formule (I) selon la revendication 1, dans lequel on soumet un composé de vitamine D3 de formule (II):

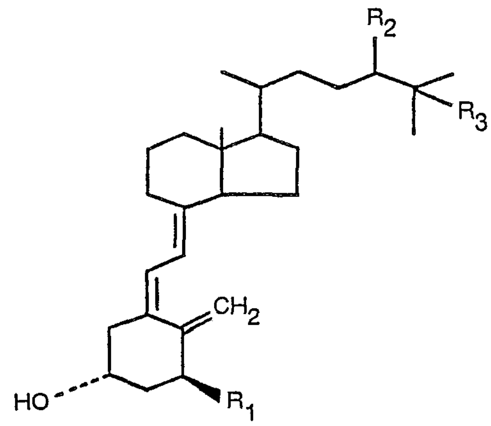

(II)

dans laquelle $R_1$, $R_2$ et $R_3$ sont tels que définis ci-dessus, à une photo-oxydation dans un solvant inerte organique.

3. Procédé selon la revendication 2, dans lequel on utilise un photosensibilisateur tel que le Rose Bengale, l'éosine ou le bleu de méthylène.

4. Procédé selon l'une des revendications 2 et 3, dans lequel le composé de vitamine D3 est illuminé avec la lumière visible d'une lampe à halogène ou d'une lampe à tungstène, en présence d'air ou d'oxygène.

5. Composé selon la revendication 1, dans lequel le dérivé de 6,19-épidioxyvitamine D3 est le 6,19-épidioxy-9,10-secocholesta-5(10),7-diène-3β,24-diol.

6. Composé selon la revendication 1, dans lequel le dérivé de 6,19-épidioxyvitamine D3 est le 6,19-épidioxy-9,10-secocholesta-5(10),7-diène-1α,3β,24-triol.

7. Composé selon la revendication 1, dans lequel le dérivé de 6,19-épidioxyvitamine D3 est le 6,19-épidioxy-9,10-secocholesta-5(10),7-diène-1α,3β,25-triol.

8. Compositions pharmaceutiques comprenant au moins un des composés selon les revendications 1 à 7.

**Revendications pour l'Etat contractant: AT**

1. Procédé pour la préparation d'un dérivé de 6,19-épidioxyvitamine D3 de formule (I)

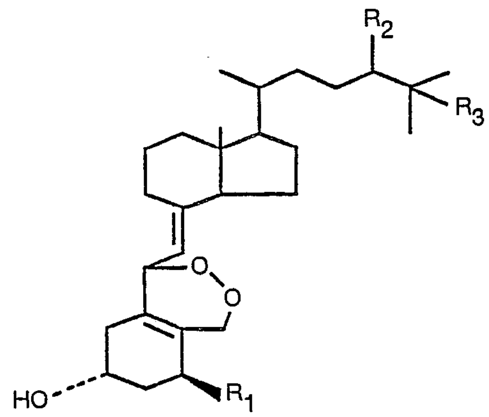

(I)

dans laquelle $R_1$, $R_2$ et $R_3$ sont chacun un atome d'hydrogène ou un groupe hydroxyle; lorsque $R_1$ est un atome d'hydrogène, $R_2$ représente un groupe hydroxyle et $R_3$ est un atome d'hydrogène ou un groupe hydroxyle; lorsque $R_1$ et $R_2$ représentent tous les deux un groupe hydroxyle, $R_3$ est un atome d'hydrogène ou un groupe hydroxyle; et lorsque $R_1$ est un groupe hydroxyle et $R_2$ est un atome d'hydrogène, $R_3$ représente un groupe hydroxyle, dans lequel procédé on soumet un composé de vitamine D3 de formule (II)

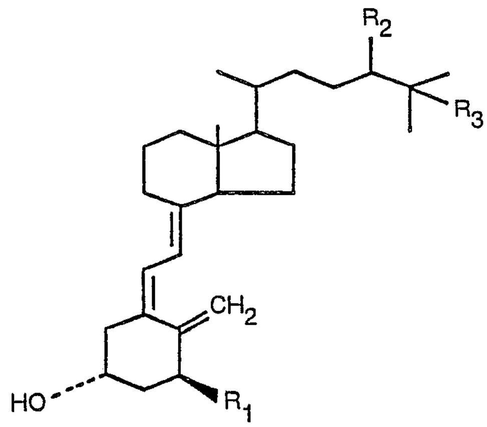

(II)

dans laquelle $R_1$, $R_2$ et $R_3$ sont tels que définis ci-dessus, à une photo-oxydation dans un solvant organique inerte.

2. Procédé selon la revendication 1, dans lequel on utilise un photosensibilisateur tel que le Rose Bengale, l'éosine ou le bleu de méthylène.

3. Procédé selon l'une des revendications 1 et 2, dans lequel le composé de vitamine D3 est illuminé avec la lumière visible d'une lampe à halogène ou d'une lampe à tungstène, en présence d'air ou d'oxygène.

4. Procédé pour préparer un composé selon la revendication 1, dans lequel le dérivé de 6,19-épidioxyvitamine D3 est le 6,19-épidioxy-9,10-secocholesta-5(10),7-diène-3β,24-diol.

5. Procédé pour préparer un composé selon la revendication 1, dans lequel le dérivé de 6,19-épidioxyvitamine D3 est le 6,19-épidioxy-9,10-secocholesta-5(10),7-diène-1α,3β,24-triol.

6. Procédé pour préparer un composé selon la revendication 1, dans lequel le dérivé de 6,19-épidioxyvitamine D3 est le 6,19-épidioxy-9,10-secocholesta-5(10),7-diène-1α,3β,25-triol.

7. Procédé pour préparer une composition pharmaceutique comprenant la formulation d'au moins un des composés selon les revendications 1 à 6.